# EUROPEAN PATENT APPLICATION

(11) **EP 4 822 626 A2**
(43) Date of publication of application: **14.10.2026**
(21) Application number: 26156473.6
(22) Date of filing: 26.10.2016
(51) Int. Cl.: G06Q 10/10, G06Q 50/22, G16H 10/20, G16H 50/20

(54) **GENERATING RTSM SYSTEMS FOR CLINICAL TRIALS**

(30) Priority: 26.10.2015 US 201562246210 P; 15.01.2016 US 201662279243 P
(62) Document number(s) of the earlier application(s) in accordance with Art. 76 EPC: 16860708.3 / 3 369 058
(71) Applicant: 4G CLINICAL LLC, Wellesley, MA 02481 (US)
(72) Inventor: TOURTELLOTTE, Edward Alan, Dover, MA 02030 (US)
(74) Representative: do Nascimento Gomes, Rui

(57) **Abstract**

A computer system for generating a Randomization and Trial Supply Management (RTSM) system includes at least one programmable processor and a computer-readable medium storing instructions that, when executed, cause the at least one programmable processor to perform operations including: receiving and editing a specification for clinical trial in a specification editor, checking and improving quality of the specification by a specification checker, presenting the specification and providing feedback to a user in a specification viewer, interpreting the specification by a specification interpreter utilizing natural language processing (NLP), building a clinical trial study based on interpretation result of the specification interpreter, and configuring an RTSM system based on the clinical trial study.

## Description

### RELATED APPLICATIONS

This application is a divisional of the European Patent Application 16860708.3, European regional phase of PCT application PCT/US2016/058913. This application claims priority to U.S. Provisional Application 62/246,210, filed on October 26, 2015, and U.S. Provisional Application 62/279,243, filed on January 15, 2016, both of which are incorporated herein in their entirety.

### TECHNICAL FIELD

The subject matter described herein relates generally to Randomization and Trial Supply Management (RTSM) systems for clinical trials, and more specifically to generating, programming, and/or configuring RTSM systems.

### BACKGROUND

Randomizing patients and correctly supplying treatments are crucial to clinical trials. Most clinical trials rely on an RTSM system to carry out critical functions of the trials, including patient randomization, blinding, drug dispensing, and automatic drug resupply. RTSM systems are sometimes also known as IVR (Interactive Voice Response), IWR (Interactive Web Response) or IRT (Interactive Response Technologies) systems.

In early days, the first generation of RTSM technology typically used patient-numbered kits, which were shipped to all clinical trial sites in full blocks corresponding to the full course of treatments. The first generation RTSM systems were inefficient and commonly caused massive waste of supply.

In the 1990s, interactive voice response (IVR) systems marked the beginning of the second generation of the RTSM technology. An IVR system usually randomized patients over the phone, and later dispensed drug and resupplied sites. Supplying treatments became more flexible. For example, patient kits could be interchangeable for other equivalent kits; the supply for a dropped-out patient could be used by a subsequent patient in the same study; and shipping no longer needed to occur in blocks, nor in full courses of treatment. In addition, IVR allowed more complex trial designs, such as ones involving variable dosing or multi stratified trials. Later, interactive web response (IWR) systems have replaced onerous phone keypad work and associated errors with easier-to-use web user interfaces for collecting, organizing, and display data.

In the 2000s, "parameter driven" or configurable RTSM systems advanced the RTSM technology to its third generation. A configurable RTSM system is designed to replace the slow and error-prone development process with faster and screen-based "configuration." In some situations, clinical trials can be set up right out of the box by selecting or entering information such as visit schedule, treatment arms, dosing, etc.

While the RTSM technology advances over time, clinical trial designs have become more and more complex and difficult to manage. Generating RTSM systems is still a time-consuming and labor-intense process. For example, a clinical trial sponsor (e.g., a pharmaceutical company or a contract research organization (CRO)) must first author a lengthy document (i.e., "specification") describing the requirements and other information of the clinical trial. An RTSM system vendor must then engage software developers to manually configure and/or program an RTSM system based on the specification. This process can take as long as 12 weeks or more. Although configurable RTSM systems usually require less programming, they only partially mitigate the onerous and lengthy process.

### SUMMARY

In accordance with the disclosed subject matter, systems and methods are described for generating, programming, and/or configuring RTSM systems for clinical trials.

Disclosed subject matter includes, in one aspect, a computer system for generating a Randomization and Trial Supply Management (RTSM) system, which includes at least one programmable processor and a computer-readable medium storing instructions that, when executed, cause the at least one programmable processor to perform operations including: receiving and editing a specification for clinical trial in a specification editor, checking and improving quality of the specification by a specification checker, presenting the specification and providing feedback to a user in a specification viewer, interpreting the specification by a specification interpreter utilizing natural language processing (NLP), building a clinical trial study based on interpretation result of the specification interpreter, and configuring an RTSM system based on the clinical trial study.

In some embodiments, in the computer system for generating an RTSM system, the specification interpreter includes a master interpreter, one or more specialized interpreters, one or more information extractors, one or more specialized NLP tools, and one or more core NLP libraries.

In some embodiments, in the computer system for generating an RTSM system, the one or more specialized interpreters include at least one of a study level interpreter, a primary event interpreter, a randomization interpreter, an action interpreter, a drug supply interpreter, and a notification interpreter.

In some embodiments, in the computer system for generating an RTSM system, the computer-readable medium storing instructions that, when executed, cause the at least one programmable processor to perform further operations including: receiving the specification at a master interpreter, dividing the specification into a plurality of themes by the master interpreter, analyzing each of the plurality of themes by one or more specialized interpreters, extracting information from each of the plurality of themes using one or more information extractors, invoking one or more specialized NLP tools by at least one of the one or more information extractors to further analyze each of the plurality of themes, and calling one or more core NLP libraries by at least one of the one or more specialized NLP tools to assist processing each of the plurality of themes.

Disclosed subject matter includes, in another aspect, a computerized method of generating a Randomization and Trial Supply Management (RTSM) system, performed by at least one programmable processor in a computer system, which includes: receiving and editing a specification for clinical trial in a specification editor, checking and improving quality of the specification by a specification checker, presenting the specification and providing feedback to a user in a specification viewer, interpreting the specification by a specification interpreter utilizing natural language processing (NLP), building a clinical trial study based on interpretation result of the specification interpreter, and configuring an RTSM system based on the clinical trial study.

In some embodiments, in the computerized method of generating an RTSM system, interpreting the specification includes: receiving the specification at a master interpreter, dividing the specification into a plurality of themes by the master interpreter, analyzing each of the plurality of themes by one or more specialized interpreters, extracting information from each of the plurality of themes using one or more information extractors, invoking one or more specialized NLP tools by at least one of the one or more information extractors to further analyze each of the plurality of themes, and calling one or more core NLP libraries by at least one of the one or more specialized NLP tools to assist processing each of the plurality of themes.

In some embodiments, in the computerized method of generating an RTSM system, the one or more specialized interpreters comprise at least one of a study level interpreter, a primary event interpreter, a randomization interpreter, an action interpreter, a drug supply interpreter, and a notification interpreter.

Disclosed subject matter includes, in yet another aspect, a Randomization and Trial Supply Management (RTSM) system generator, executed by at least one programmable processor in a computer system, which includes: a specification editor for receiving and editing a specification for a clinical trial, a specification viewer for presenting the specification and providing feedback to a user, a specification checker for checking and improving quality of the specification, a specification interpreter configured to utilize natural language processing (NLP) to interpret the specification, and a study builder configured to build a clinical trial study based on output of the specification interpreter and to configure an RTSM system based on the clinical trial study.

In some embodiments, in the RTSM system generator, the specification interpreter includes: a master interpreter, one or more specialized interpreters, one or more information extractors, one or more specialized NLP tools, and one or more core NLP libraries.

In some embodiments, in the RTSM system generator, the one or more specialized interpreters comprise at least one of a study level interpreter, a primary event interpreter, a randomization interpreter, an action interpreter, a drug supply interpreter, and a notification interpreter.

Disclosed subject matter includes, in yet another aspect, a Randomization and Trial Supply Management (RTSM) system with a built-in forecaster, which includes: at least one programmable processor and a computer-readable medium storing instructions that, when executed, cause the at least one programmable processor to perform operations, which include: receiving at least one supply chain control levels via a user interface of the RTSM system from a user, adjusting configuration of the RTSM system based on the at least one supply chain control levels, and presenting adjustment results to the user dynamically.

In some embodiments, in the RTSM system, the at least one supply chain control levels include a first confidence level for unpredictable demand of known and unknown patients, and the user interface presents a first user interface (UI) element to receive the first confidence level from the user.

In some embodiments, in the RTSM system, the at least one supply chain control levels include a second confidence level for demand of patients with a partially known schedule, and the user interface presents a second user interface (UI) element to receive the second confidence level from the user.

In some embodiments, in the RTSM system, the at least one supply chain control levels include a number for a long prediction window, and the user interface presents a third user interface (UI) element to receive the number for the long prediction window.

In some embodiments, in the RTSM system, the forecaster utilizes at least one statistical distributions to perform forecasting.

In some embodiments, in the RTSM system, the at least one statistical distributions comprise Poisson distribution.

Articles of manufacture are also described that comprise computer executable instructions non-transitorily stored on computer readable media, which, when executed by a computer, causes the computer to perform operations herein. Similarly, computer systems are also described that may include a processor and a memory coupled to the processor. The memory may temporarily or permanently store one or more programs that cause the processor to perform one or more of the operations described herein.

Various embodiments of the subject matter disclosed herein can provide one or more of the following features. An RTSM system can be generated and configured automatically and rapidly, e.g., from a clinical trial specification. The clinical trial specification can be in a text format and written in a natural language (e.g., English). An RTSM system generator can receive the clinical trial specification from, e.g., a clinical trial sponsor such as a pharmaceutical company and automatically interpret the specification using natural language processing. The RTSM system generator can build a clinical trial study based on the result of the natural language processing of the specification and generate/configure a RTSM system automatically, with minimal or no human intervention. The lengthy and onerous process of manually programming a RTSM system (e.g., by a human programmer) can be avoided. This can lead to significant savings on time and labor during initial RTSM system setup. Interpreting clinical trial specifications via natural language processing can also help insulate the users from future clinical trial complexities.

Various embodiments of the subject matter disclosed herein can also provide one or more of the following features. An RTSM system can contain a built-in "forecaster" to provide resupply and forecasting functionality. The RTSM system can take advantage of the fact that it has a forecaster at its heart in order to figure out and set appropriate detailed parameters such as buffer values throughout the supply chain. The RTSM system can provide user interface (e.g., dials) that allows users to adjust certain parameters (e.g., supply chain control or confidence levels, long window period, etc.) of the RTSM system. In response, the RTSM system can be configured automatically and instantly based on the user input. The corresponding changes to the RTSM system can be presented to the users instantly.

The details of one or more variations of the subject matter described herein are set forth in the accompanying drawings and the description below. Other features and advantages of the subject matter described herein will be apparent from the description and drawings, and from the Clauses and claims.

### DESCRIPTION OF DRAWINGS

FIG. 1 illustrates an RTSM system according to some embodiments of the subject matter described herein;
FIG. 2 illustrates an RTSM system generator according to some embodiments of the subject matter described herein;
FIG. 3 describes in detail the Specification Interpreter of the RTSM system generator illustrated in FIG. 2.
FIG. 4 illustrates an RTSM system core according to some embodiments of the subject matter described herein;
FIG. 5 describes in detail the Resupply and Forecasting Module of the RTSM system core illustrated in FIG. 4.
FIG. 6 illustrates an RTSM system generating process according to some embodiments of the subject matter described herein;
FIG. 7 describes in detail the interpreting specification step of the RTSM system generation process illustrated in FIG. 6;
FIGS. 8A-8C contain screenshots of certain sample user interfaces according to some embodiments of the subject matter described herein;
FIG. 9 illustrates an RTSM system generating platform according to some embodiments of the subject matter described herein.

Like reference symbols in the various drawings indicate like elements.

### DETAILED DESCRIPTION

An RTSM system specification can be a document in any format (e.g., plain text), describing the requirements and other related information defining randomization and supply management (among other functions) of a clinical trial. An RTSM system specification is generally considered complete if it contains sufficient information (e.g., logic and description) to generate an RTSM system. For example, a complete RTSM system specification contains enough information to allow vendor programmers to program and/or configure an RTSM system. The subject matter described herein discloses a technology via which an RTSM system specification can be created and self-sufficient to set up an RTSM system, without the need for programming or configuration. Via this technology disclosed herein, the initial RTSM system setup can essentially become automated and instantaneous.

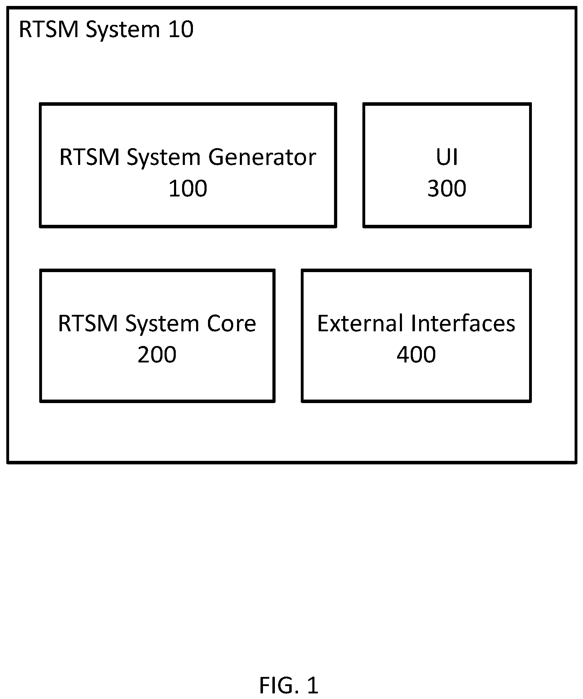

FIG. 1 illustrates an RTSM system 10 according to some embodiments of the subject matter disclosed herein. The RTSM system 10 illustrated in FIG. 1 can include an RTSM system generator 100, a RTSM system core 200, user interfaces (UI) 300, and external interfaces 400. The RTSM system 10 can further include additional components.

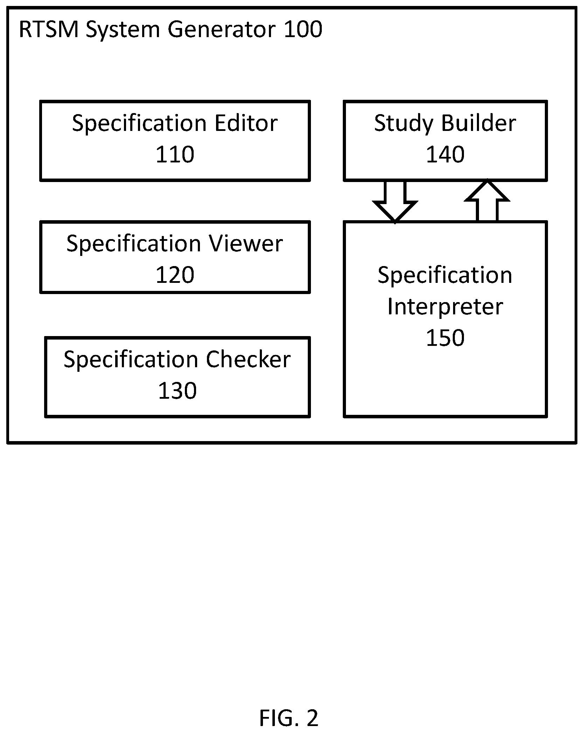

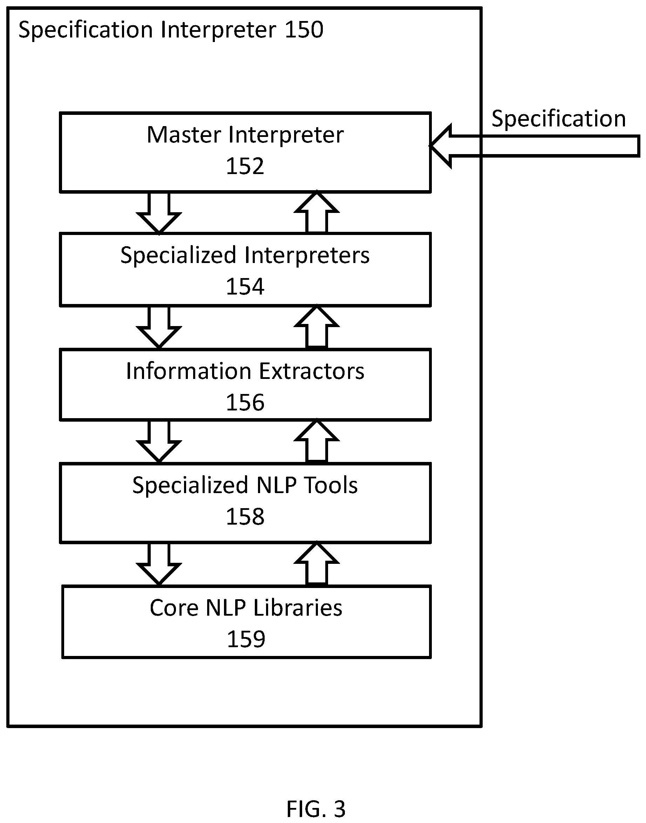

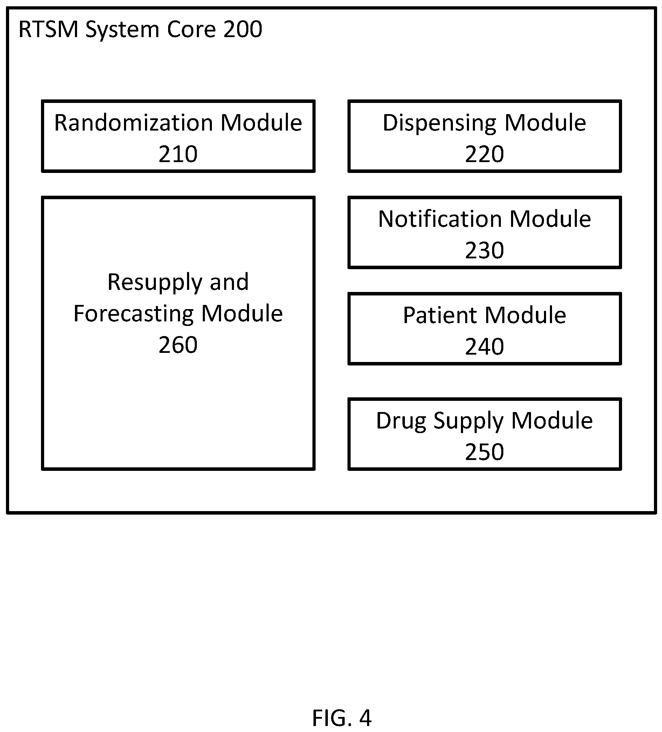

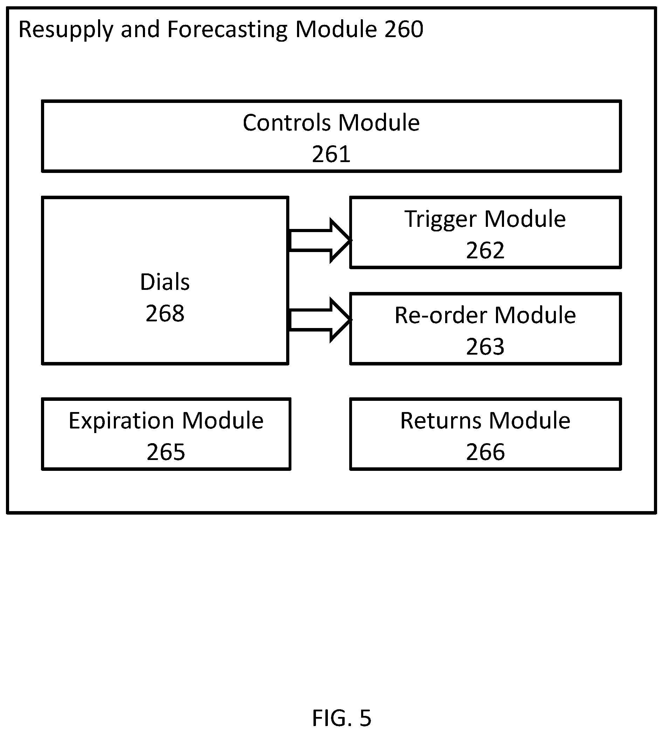

The RTSM system generator 100 can generate configurations, logics, and data structures for a clinical trial and generate an instance of the RTSM system core 200. The RTSM system generator 100 will be described in greater detail below along with FIGS. 2 and 3. The RTSM system core 200 will be described in greater detail below along with FIGS. 4 and 5. The UI 300 can provide user interfaces to the RTSM system 10 for various audiences. For example, the UI 300 can include an end user UI. The end user UI can provide a user interface for end users, such as a clinical trial sponsor (e.g., a pharmaceutical company), a patient, a hospital, and a doctor, etc. The UI 300 can also include an admin user UI. The admin user UI can provide a user interface for an administration user of the RTSM system, e.g., to configure, fine-tune, or modify data in the RTSM system. The UI 300 can also include user interfaces for other audiences. The external interfaces 400 can provide the RTSM system 10 interfaces for input/output from/to other external systems, such as shipping vendors, CROs system, EDC (Electronic Data Capture) systems, and ERP (Enterprise Resource Planning) systems. The external interfaces 400 can also provide notifications to external systems.

FIG. 2 illustrates an RTSM system generator 100 according to some embodiments of the subject matter disclosed herein. The RTSM system generator 100 illustrated in FIG. 2 can include a specification editor 110, a specification viewer 120, a specification checker 130, a study builder 140, and a specification interpreter 150. The RTSM system generator 100 can further include additional components.

The specification editor 110 can receive and edit a specification, e.g., from a user. The specification editor 110 can be in the form of a plain language (e.g., English) editor. A specification writer (e.g., an expert configuring RTSM for a clinical trial) can input the draft specification into the specification editor 110. For example, the specification writer can type in or import descriptive text, or import/construct matrices in the specification editor 110. The descriptive text and the matrices can describe the functions and features of a desired RTSM system for a clinical trial. The specification editor 110 can provide comprehensive facilities to the specification writer to improve productivity in drafting and creating the specification. For example, the specification editor 110 can provide a set of useful toolboxes, keyword color coding, and keyword completion.

The specification viewer 120 can present the specification and provide feedback, e.g., to the user. The specification checker 130 can check and improve the quality of the specification. The specification checker 130 can check and verify the specification entered by the specification writer. For example, the specification checker 130 can perform spelling checking, examine the grammar and syntax, and/or check for inconsistent, unclear or conflicting terms/phrases/sentences. The specification checker 130 can also suggest correct spelling and alternative terms/phrases/sentences. In addition, the specification checker 130 can help ensure that the specification writer draft the specification within certain limitations of logic and correctly use certain keywords. For example, the specification checker 130 can provide live checking to flag potential errors and highlight certain keywords while the specification writer enters the descriptive text into the specification editor 110. The specification checker 130 can warn the specification writer of any potential defects in the draft specification and assist the specification writer to improve the specification, e.g., by suggesting corrections any potential errors or suggesting/enforcing usage of consistent keywords. The specification viewer 120 can provide feedback, e.g., warning/error messages or suggested changes, to the user. As a result, the specification editor 110, along with the specification viewer 120 and the specification checker 130, can create a complete RTSM system specification with well-formatted contents detailing the requirements and other related information of a target RTSM system. The RTSM system specification, e.g., created by the specification editor 110, can be passed into the study builder 140 to build a clinical trial RTSM.

The study builder 140 can automatically and quickly generate configurations, logic, and data structures for a clinical trial study based on the RTSM system specification and use them to generate an instance of the RTSM system core 200. The study builder 140 can invoke the specification interpreter 150 to interpret the specification received from the specification editor 110. The specification interpreter 150 can understand and interpret the content of the received RTSM system specification. In some situations, the RTSM system generator 100 can generate a development or test RTSM system. Vendors or sponsors can test-run the development or test RTSM system to check and verify that it fits their needs and goals. The vendors or sponsors can then request modifications to the RTSM system specification (e.g., via the specification editor 110) followed by another run of the study builder 140 to re-generate an updated RTSM system. Once the test RTSM system is verified or approved, it can become a production RTSM system, which is ready to receive data loads (e.g., list of users, clinical trial sites, materials, randomization blocks, etc.) and begin conducting the clinical trial.

In some embodiments, the specification editor 110 and the specification viewer 120 can be considered the front end of the RTSM system generator 100. For example, the specification editor 110 can receive the user input; the specification viewer 120 can provide feedback to the users. In contrast, the study builder 140 and the specification interpreter 150 can be considered the back end of the RTSM system generator 100. For example, the specification interpreter 150 can interpret the specification and generate logics for the target RTSM system. The specification interpreter 150 can perform functions that are transparent to the users.

In some embodiments, the study builder 140 and the specification interpreter 150 can be configured to execute automatically after the specification editor 110 finishes creating the specification. Thus, specification creation and interpretation can be performed as a single continuous task, eliminating the need for programming and configuring an RTSM system.

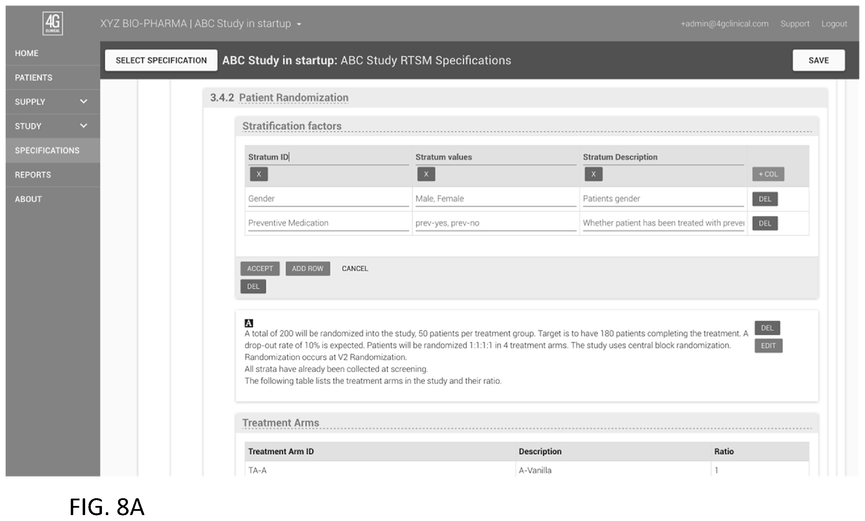

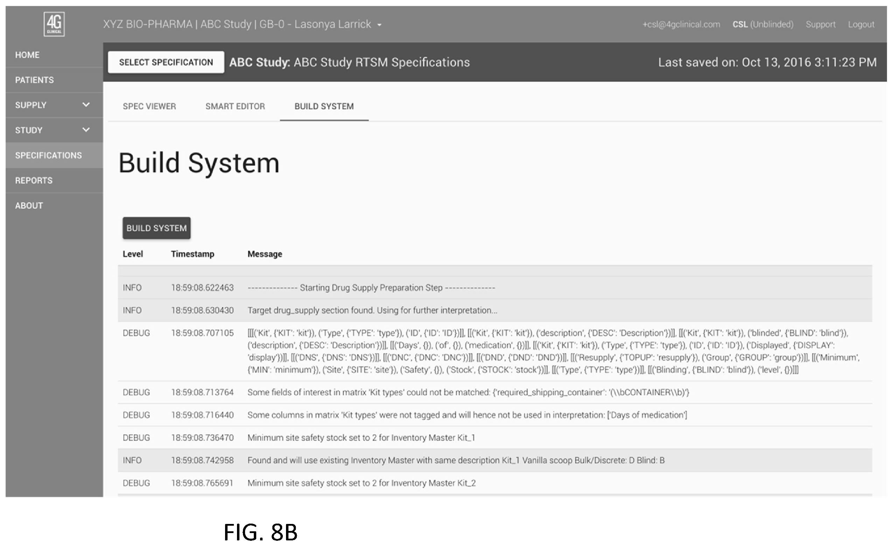

FIGS. 8A-8B contain screenshots of certain sample user interfaces illustrating, e.g., the specification editor 110 and the specification viewer 120.

FIG. 3 describes in detail the specification interpreter 150 of the RTSM system generator 100. The specification interpreter 150 illustrated in FIG. 3 can include a master interpreter 152, one or more specialized interpreters 154, one or more information extractors 156, one or more specialized natural language processing (NLP) tools 158, and one or more core NLP libraries 159. The specification interpreter 150 can further include additional components.

A core NLP library, such as the core NLP libraries 159, can include information of a natural language (e.g., English), such as vocabulary and syntax, corpora, taggers, tokenizers, stemmers, lemmatizers, learning algorithms and more. The core NLP library 159 can be used by the specification interpreter 150 to examine the specification entered by a specification writer. For example, the specification interpreter 150 can rely on the core NLP library 159 to recognize words and stems, flag incorrect spelling, analyze grammar and meaning, detect logical operations and data definitions, and/or suggest alternative terms. In some situations, the core NLP library 159 can be provided by a third party, such as from certain programming language (e.g., Java or Python) development toolkits (e.g., Natural Language Tool Kit or NLTK).

A specialized NLP tool, such as the specialized NLP tools 158, can provide system-specific extensions to the core NLP library 159. For example, the specialized NLP tools 158 can include additional information for words, phrases, terms, syntax, reserved words/phrases, keywords, and operators, for example as may be commonly used in the context of certain fields (e.g., clinical trials). The specialized NLP tool 158 can also include advanced language analysis functionality, such as tokenizing, stemming, grammar, collocations, language corpus, sentence structure and meaning analysis, among other natural language processing features. The specialized NLP tools 158 can also include artificial neural networks.

The specialized NLP tools 158 can also be used by the specification interpreter 150 to examine the specification entered by a specification writer. For example, the specification interpreter 150 can rely on the specialized NLP tools 158 to recognize system-specific words, flag incorrect spelling, and/or suggest alternative terms. The specialized NLP tools 158 can be provided by a third party or internally developed.

An information extractor, such as the information extractors 156, can extract certain information from the specification. One type of information extracted can include matrices (e.g., information structured in a tabular manner). A matrix may be used in specification development when it is more convenient to organize data in matrix form than in natural language. For example, a matrix can be used to define patient visit schedules and associated dosing regimens or other visit events; a matrix can also be used to define patient kit types, supply chain relationships and lead times, or any other data which is naturally defined in a matrix. A matrix can be hand-entered by an expert user, provided by a third party or internally developed and made available in the RTSM system generator 100. A matrix can be used to ease and speed up specification drafting. Another type of information extracted can include certain style of sentences such as definitions. A definition could for example be structured as [SUBJECT OF DEFINITION] [VERB PROPOSITION] [ITEM or LIST OF ITEMS], but structure could differ or be made more flexible to enable use of free English style in writing and yet extracting relevant information. Other styles of sentences can be defined as necessary.

The master interpreter 152 can receive an RTSM system specification. The master interpreter 152 can perform top level analysis of the specification and can organize interpretation in a logical sequence of themes. A theme can include one or more defined RTSM topics, which can run across one or more sections of the specification document. A particular theme of the interpretation can correspond to a specialized interpreter, configured to analyze information relevant to the particular theme across the specification document (in one or more sections). The master interpreter 152 can invoke different specialized interpreters to process different themes of the RTSM generation through the specification.

The specialized interpreters 154 can include a study level interpreter, a primary event interpreter, a randomization interpreter, an action interpreter, a drug supply interpreter, a notification interpreter, or other interpreters as required by the RTSM requirements. A study level interpreter can interpret the high level information in the specification. For example, high level information can include certain study level information, such as the total number of patients desired to be screened and randomized, the randomization scheme and method, and other required parameters. A primary event interpreter can interpret primary event information in the specification. For example, a primary event can include a visit schedule. A randomization interpreter can interpret randomization information in the specification. An action interpreter can interpret event actions information in the specification. For example, an event action can include information such as what needs to be done within the context of each patient visit. A drug supply interpreter can interpret the drug supply information in the specification. For example, the drug supply information can include supply network, shipping rules, dispensing kits info, etc. A notification interpreter can interpret notification information in the specification. For example, a notification can include when and how to notify a user about a study event such a screening or randomization of a patient. The list of specialized interpreters is not exhaustive. Additional specialized interpreters can be added when needed.

In some situations, the master interpreter 152 can be an entry point into the specification interpreter 150. For example, the master interpreter 152 can receive a specification and divide the interpretation into different sections or themes. The master interpreter 152 can then invoke an appropriate specialized interpreter 154 to analyze each section or theme. Each specialized interpreter 154 can invoke one or more information extractors 156, as needed, to extract information from each section or theme of the specification, such as matrices and definitions. Each information extractor 156 can invoke one or more specialized NLP tools 158, as needed. Each specialized NLP tools 158 can then invoke one or more core NLP libraries to assist processing each section or theme of the specification.

The output result of a core NLP library 159 can be returned to the specialized NLP tool that invokes the core NLP library. The output result of a specialized NLP tool 158 can be returned to the information extractor that invokes the specialized NLP library. The output result of an information extractor 156 can be returned to the specialized interpreter that invokes the information extractor. The output result of a specialized interpreter 154 can be returned to the master interpreter that invokes the specialized interpreter.

In some embodiments, the output of the specification interpreter 150 can include basic data structures and driving logic. The basic data structures and driving logic can include logic containers and other necessary or related structures for the target RTSM system. For example, the data structures may hold planned patient visit and dosing information, collectible identifying information (e.g. initials, date of birth), data driving information (e.g. BMI for use in dosing), data related to supply chain organization, supply shipments and/or patient kits, or any other data which is desired to be collected or acted upon during the trial. Where necessary, logic containers can hold specific processing instructions for the RTSM in order to comply with the specification.

One feature of RTSM systems is their ability to predict demand of clinical supply at investigative sites where patients are participating in clinical trials, and to pre-seed those sites with appropriate drug for dispensing. This function is typically called a "resupply algorithm." Resupply algorithms can be controlled by parameters such as safety stock minimum (or floor-ceiling values), look-ahead windows, and more. The management of these parameters - figuring out what they should be across the world in a given clinical trial, then keeping track of and updating them - is a burden for pharmaceutical companies.

In the past decade, a few forecasting tools have been developed in order to help plan supply needs in advance, and also to tune resupply parameters (and get a view of what is coming) in an ongoing basis in a clinical trial. Some of these forecasting tools use simulation techniques as the backbone of their forecasting power. Since forecasting tools need to approximate the behavior of the RTSM resupply algorithm which they are forecasting for, they generally need to have the same or similar parameters as a number of different RTSM offerings, and rarely if ever can they match the RTSM resupply algorithm accurately.

Resupply algorithms (and forecasters) generally deal with two types of demand: predictable demand and unpredictable demand. Predictable demand encompasses requirements which will be consumed by existing, known patients at any time in the future, where the treatment type and dosage level is known in advance. Unpredictable demand encompasses requirements which will be consumed either by unknown (non-existing) patients ("uncorrelated unpredictable demand"), either by known patients who have not yet had a treatment assigned through randomization, or by known patients who have a general course of treatment assigned but the study design itself includes variability in future dosing ("correlated unpredictable demand").

Competent RTSM forecasters generally deal appropriately with predictable demand, but only the most robust and powerful forecasters can deal with unpredictable demand, which is by its nature unknown in advance. In the best case, simulators yield statistically realistic upper limits on unpredictable demand over some window in time, and the result is used to create safety stock or floor-ceiling buffers (stock placed at sites in advance without knowing if it will be used.) The process of managing these buffers and settings is currently always manual, and labor intensive.

Two time horizons are important to RTSM. The first is sometimes known as the "short window" and is usually equal to or slightly greater than the shipping lead time from a supplying depot into a site in question. For example, buffer or safety stocks can be set to cover for all or almost all anticipated needs within this window of time, and hence prevent stock-outs or missed patient dispensing. Because it may be undesirable to ship replenishment every time a drug is consumed, a second window, sometimes called the "look-ahead window" or "long window," tells the system how long inventory should hold before another shipment is needed. For example, a long window setting might be 30 days.

In some situations, good forecasting tools can stay in sync with good RTSM systems (via regular data feeds) and can continue to provide advice for sponsor companies to set parameter values as described above. In other situations, however, this does not happen. The complexity of many aspects of this process, all centered on deep understanding of resupply algorithms and their parameters, overwhelms many clinical supply professionals (or others responsible for this function).

FIG. 4 illustrates an RTSM system core 200 according to some embodiments of the subject matter described herein. The RTSM system core 200 illustrated in FIG. 4 can include a randomization module 210, a dispensing module 220, a notification module 230, a patient module 240, a drug supply module 250, and a resupply and forecasting module (a "forecaster") 260. The RTSM system core 200 can further include additional components.

The randomization module 210 can determine whether a particular patient can be allocated a treatment arm or not, and perform that allocation when possible, following certain randomization algorithms - of which many variants exist. The dispensing module 220 can determine which / how many drug kits (and other items such as ancillaries) can be given to a patient coming for a certain visit. The notification module 230 can watch over a number of patient or supply chain-related events (e.g. patient randomized, shipment sent...) and apply opt-in and opt-out rules to determine who to send notifications to and in which format or formats. The patient module 240 can handle all patient-related requirements, such as moving a patient along his/her visit schedule, changing his/her status, moving him/her to a different site, among other things. The drug supply module 250 can handle lot management and shipment-related features, as well as quarantining, temperature control reporting and actions, among other things.

The subject matter disclosed herein can provide resupply and forecasting features to RTSM systems. According to some embodiments of the disclosed subject matter, a forecaster with the "forecasting" function, such as the resupply and forecasting module 260, can be built into the heart of an RTSM system. The same forecaster, which runs pre-study and mid-study forecasts, can be used for nightly resupply algorithm calculations and shipment requests. In this way, there is, by design, no divergence between forecaster and resupply algorithms and parameters. The RTSM system, according to some embodiments of the disclosed subject matter, can take advantage of the fact that it has a forecaster at its heart in order to automatically figure out and use appropriate required stock values throughout the supply chain.

In addition, the RTSM systems, according to some embodiments of the disclosed subject matter, can replace (in full or in part) simulation as a forecasting technique with an exact statistical representation of demand variability, which can be more accurate and give faster results. For example, uncorrelated unpredictable demand, generated by projected (unknown) patients, can be accounted for via a simple projected "patients per month" value and can be modelled as random arrival process, such as the Poisson distribution.

FIG. 5 describes in detail the resupply and forecasting module 260 (or the "forecaster") of the RTSM system core 200 illustrated in FIG. 4. The resupply and forecasting module 260 illustrated in FIG. 5 can include a controls module 261, a trigger module 262, a re-order module 263, an expiration module, a returns module 266, and one or more dials 268. The resupply and forecasting module 260 can further include additional components.

A controls module 261 can list and check all resupply and forecasting parameters which will drive the supply chain resupply algorithms. A trigger module 262 can determine, on a particular moment and for a particular site, whether inventory is sufficient to cover the short-term needs, or whether a new order should be placed to replenish inventory. A re-order module 263 can determine how much should be re-ordered for a site to cover for its long(er) term needs. An expiration module can determine adequateness of lots and kits, and pick appropriate kits that will meet supply chain timing (e.g. Do Not Ship date) and expiration requirements for anticipated demand. A returns module 266 can help with the backwards supply chain flow in determining how remaining/expired kits should be handled at sites and depots, and track data for accountability.

The dials 268 can include different types of dials, such as supply chain control or confidence dial, long window dial, etc. The resupply and forecasting module 260 can contain zero or more dials of each type. In some embodiments, the resupply and forecasting module 260 can provide a "risk dial" via their user interface. A sponsor or other responsible party can use the supply chain control dial to set a level of confidence at which, for example, the total projected demand (e.g., predictable, correlated unpredictable, uncorrelated unpredictable, etc.) should be supported. A user can move the dial back and forth over different confidences and view the resulting demand instantly on the user interface (e.g., via a web browser). The user can then present this clear choice to their own management, for example: "it will cost us $100,000 more to operate at 99% confidence rather than 95% confidence, so that we can support on average 3 patients per month in this country." In some embodiments, the forecaster can use statistical distributions (e.g., Poisson distribution), statistical methods, and mathematical techniques (e.g., simulations, symbolic computation, and function approximation, etc.) combined in a custom manner, and pre-run all forecasts for the various confidence levels, so that results may be instantly viewed as the dial(s) are turned.

In some embodiments, the resupply and forecasting module 260 can provide a second supply chain control dial via its user interface. The second supply chain control dial, e.g., for expert users, can further control the confidence on, for example, correlated unpredictable demand (e.g., related to known patients). The second supply chain control dial can be set at a higher value than the first supply chain control dial, so as to guarantee an even better service level to patients who are already known by the system. For this second supply chain control dial and related calculations, the RTSM systems can use similar statistical distributions (e.g., Poisson distribution), statistical methods, and mathematical techniques (e.g., simulations, symbolic computation, function approximation, etc.) combined in a custom manner, and pre-run all forecasts for the various confidence levels, so that results may be instantly viewed as the dial is turned.

In some embodiments, the resupply and forecasting module 260 can provide a third dial via its user interface. For example, the third dial can be the "long window dial" or "look-ahead window dial." A forecasting user can turn the long window dial to different notches and view the total increased/decreased demand based on covering supply needs for more or less time in the future. In some examples, this long window dial can also contain an automatic optimization component.

According to some embodiments of the disclosed subject matter, use of the confidence and long window dials, and resultant demand, within an RTSM system, for supply analysis and management signoff at a sponsor can provide a new approach to both forecasting and resupply algorithm control parameters. In some embodiments, combinations of multiple dials can be forecast in advance when a user is using forecasting dials for analysis. In some embodiments, the number and types of dials can increase or decrease, in order to meet various objectives, such as to give users simple means of control and visibility over cost, risk, service level, while crafting precise mathematical or statistical models in the system, or to offer an unmatched efficiency of the automated inventory management /resupply algorithms provided by RTSM systems, etc.

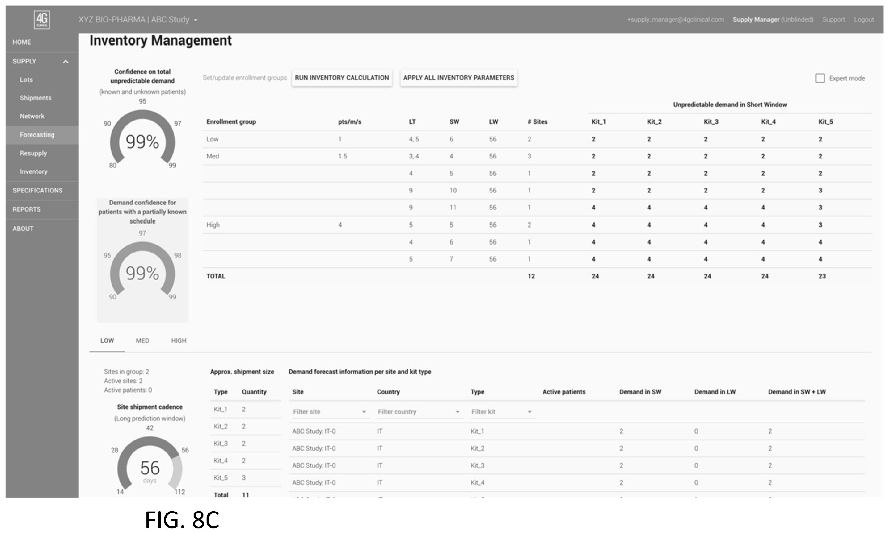

FIG. 8C contains a screenshot of certain sample user interface illustrating, e.g., the different dials, such as a first supply chain control dial, a second supply chain control dial, and a long window dial.

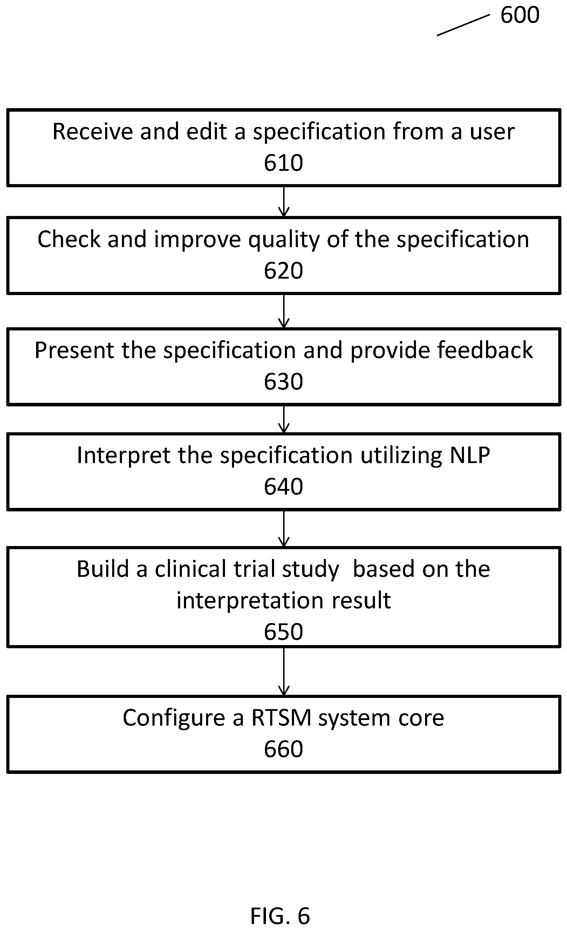

FIG. 6 illustrates an RTSM system generating process 600 according to some embodiments of the subject matter disclosed herein. At 610, an RTSM system generator, such as the RTSM system generator 100 illustrated in FIG. 2, receives and edits a specification, e.g., input by a user. The user can be a specification writer, e.g., an expert designing a clinical trial. The specification can be received and edited in the specification editor 110. At 620, the RTSM system generator 100 checks and improves the quality of the specification. The checking and improvement can be provided by the specification checker 130. At 630, the RTSM system generator 100 presents the specification and provides feedback, e.g., to a user. The presentation and feedback can be provided by the specification viewer 120. At 640, the RTSM system generator 100 interprets the specification utilizing natural language processing (NLP). The interpretation can be performed by the specification interpreter 150. At 650, the RTSM system generator 100 builds a clinical trial RTSM study based on the interpretation result of the specification interpreter. The building of the clinical trial RTSM study can be performed by the study builder 140. At 660, the RTSM system generator 100 configures an RTSM system core based on the clinical trial study.

Certain steps in the RTSM system generating process 600 can be re-ordered; one or more steps can be omitted; and additional step(s) can be added. In some embodiments, the RTSM system generating process 600 can be executed by the RTSM system generator 100 in FIG. 2.

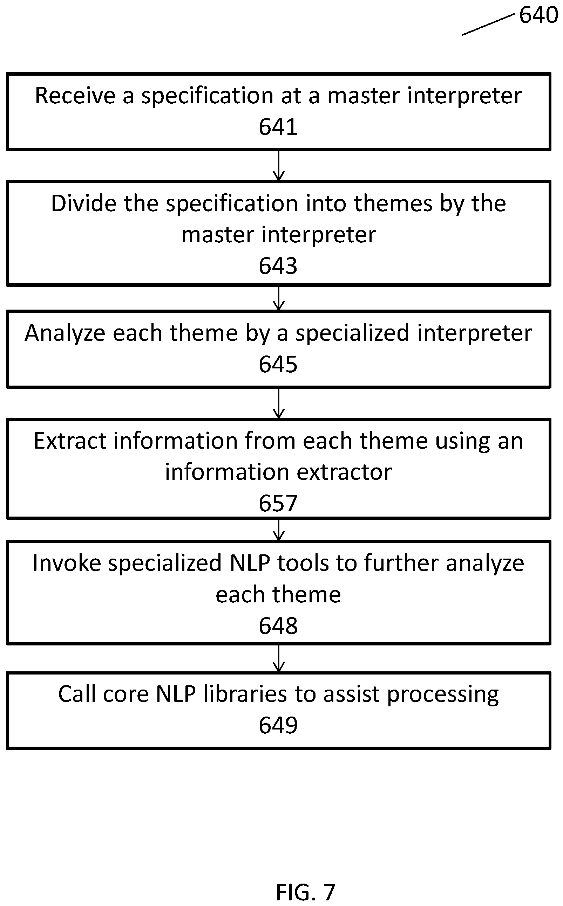

FIG. 7 describes in detail the interpreting specification step 640 of the RTSM system generating process 600 illustrated in FIG. 6. At 641, the specification interpreter 150 receives a specification. The specification can be received at the master interpreter 152. At 643, the specification interpreter 150 divides the specification into multiple themes. The division can be performed by the master interpreter 152. At 645, the specification interpreter 150 analyzes each theme within the specification. The analysis of each theme can be performed by one or more specialized interpreters 154. The one or more specialized interpreters 154 can be invoked by the master interpreter 152, e.g., based on the characteristics of each theme. At 657, the specification interpreter 150 extracts information relevant to each theme. The extraction can be performed by one or more information extractors 156. The one or more information extractors 156 can be invoked by the corresponding specialized interpreter 154, e.g., based the nature or type of the information to be extracted. At 658, the specification interpreter 150 invokes specialized NLP tools to further analyze input relevant to each theme. The one or more specialized NLP tools 158 can be invoked by the corresponding information extractor 156, e.g., based the nature or type of the natural language fragments to be analyzed. At 659, the specification interpreter 150 calls core NLP libraries to assist further processing of each section or theme. The one or more core NLP libraries 159 can be invoked by the corresponding specialized NLP tool 158, e.g., based the nature or type of natural language fragments to be analyzed.

Certain steps in the interpreting specification step 640 can be re-ordered; one or more steps can be omitted; and additional step(s) can be added. In some embodiments, the interpreting specification step 640 can be executed by the specification interpreter 150 in FIG. 3.

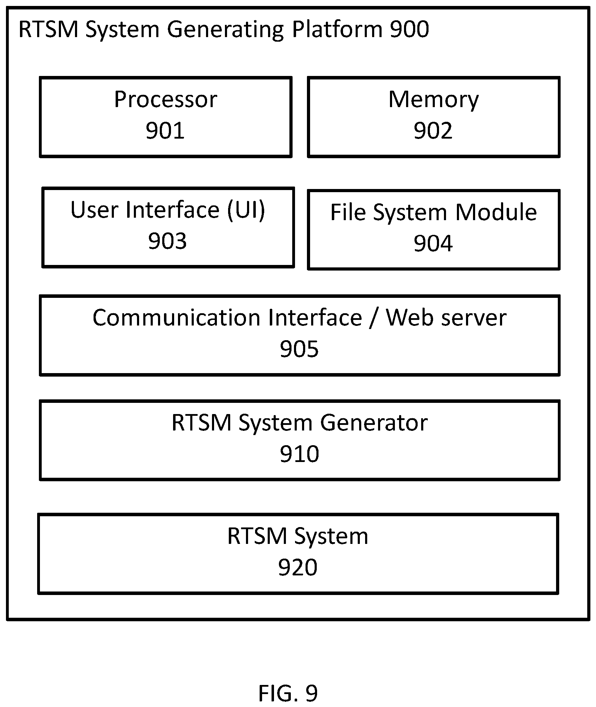

FIG. 9 illustrates a block diagram of an exemplary RTSM system generating platform 900 according to some embodiments of the disclosed subject matter. The RTSM system generating platform 900 can include at least one processor 901 and at least one memory 902. The processor 901 can be hardware that is configured to execute computer readable instructions such as software. The processor 901 can be a general processor or be an application specific hardware (e.g., an application specific integrated circuit (ASIC), programmable logic array (PLA), field programmable gate array (FPGA), or any other integrated circuit). The processor 901 can execute computer instructions or computer code to perform desired tasks. The memory 902 can be a transitory or non-transitory computer readable medium, such as flash memory, a magnetic disk drive, an optical drive, a programmable read-only memory (PROM), a read-only memory (ROM), a random access memory (RAM), or any other memory or combination of memories. Both hardware and software can be located either in proprietary facilities, or hosted by a third party with or without direct access to the physical hardware location, e.g., in a cloud computing environment.

The RTSM system generating platform 900 can also include a user interface (UI) 903, a file system module 904, and a communication interface 905. The UI 903 can provide an interface for users to interact with the RTSM system generating platform 900 in order to generate, program, configure, and/or execute an RTSM system. The file system module 904 can be configured to maintain a list of all data files, including both local data files and remote data files, in folders in a file system. The file system module 904 can be further configured to coordinate with the memory 902 to store and cache files/data. The communication interface 905 can allow the RTSM system generating platform 900 to communicate with external resources (e.g., a network or a remote client/server) or users. In some embodiments, the communication interface 905 can include a web server, which can provide a web interface to the users of the RTSM system generating platform 900. The RTSM system generating platform 900 can also include an RTSM system generator 910 and an RTSM system 920. The structure and functionality of the RTSM system generator 910 can be found earlier in this disclosure describing those of the RTSM system generator 100. The structure and functionality of the RTSM system 920 can be found earlier in this disclosure describing those of the RTSM system 10. The RTSM system generating platform 900 can include additional modules, fewer modules, or any other suitable combination of modules that perform any suitable operation or combination of operations.

One or more components in the RTSM system generating platform 900 illustrated in FIG. 9 can be omitted; additional components(s) can be added. FIG.9 is a conceptual illustration of the RTSM system generating platform 900. Various components of the RTSM system generating platform 900 can positioned locally to each other or distributed among multiple locations (e.g., across a network).

In accordance with the disclosed subject matter, a set of clauses 1-17 follows
Clause 1. A computer system for generating a Randomization and Trial Supply Management (RTSM) system, comprising:
   at least one programmable processor; and
   a computer-readable medium storing instructions that, when executed, cause the at least one programmable processor to perform operations comprising:
      receiving and editing a specification for clinical trial in a specification editor;
      checking and improving quality of the specification by a specification checker;
      presenting the specification and providing feedback to a user in a specification viewer;
      interpreting the specification by a specification interpreter utilizing natural language processing (NLP);
      building a clinical trial study based on interpretation result of the specification interpreter; and
      configuring an RTSM system based on the clinical trial study.
Clause 2. The computer system for generating an RTSM system of Clause 1, wherein the specification interpreter comprising:
   a master interpreter;
   one or more specialized interpreters;
   one or more information extractors;
   one or more specialized NLP tools; and
   one or more core NLP libraries.
Clause 3. The computer system for generating an RTSM system of Clause 2, wherein the one or more specialized interpreters comprise at least one of a study level interpreter, a primary event interpreter, a randomization interpreter, an action interpreter, a drug supply interpreter, and a notification interpreter.
Clause 4. The computer system for generating an RTSM system of Clause 1, wherein the computer-readable medium storing instructions that, when executed, cause the at least one programmable processor to perform further operations comprising:
   receiving the specification at a master interpreter;
   dividing the specification into a plurality of themes by the master interpreter;
   analyzing each of the plurality of themes by one or more specialized interpreters;
   extracting information from each of the plurality of themes using one or more information extractors;
   invoking one or more specialized NLP tools by at least one of the one or more information extractors to further analyze each of the plurality of themes; and
   calling one or more core NLP libraries by at least one of the one or more specialized NLP tools to assist processing each of the plurality of themes.
Clause 5. The computer system for generating an RTSM system of Clause 4, wherein the one or more specialized interpreters comprise at least one of a study level interpreter, a primary event interpreter, a randomization interpreter, an action interpreter, a drug supply interpreter, and a notification interpreter.
Clause 6. A computerized method of generating a Randomization and Trial Supply Management (RTSM) system, performed by at least one programmable processor in a computer system, comprising:
   receiving and editing a specification for clinical trial in a specification editor;
   checking and improving quality of the specification by a specification checker;
   presenting the specification and providing feedback to a user in a specification viewer;
   interpreting the specification by a specification interpreter utilizing natural language processing (NLP);
   building a clinical trial study based on interpretation result of the specification interpreter; and
   configuring an RTSM system based on the clinical trial study.
Clause 7. The computerized method of generating an RTSM system of Clause 6, wherein interpreting the specification comprises:
   receiving the specification at a master interpreter;
   dividing the specification into a plurality of themes by the master interpreter;
   analyzing each of the plurality of themes by one or more specialized interpreters; extracting information from each of the plurality of themes using one or more information extractors;
   invoking one or more specialized NLP tools by at least one of the one or more information extractors to further analyze each of the plurality of themes; and
   calling one or more core NLP libraries by at least one of the one or more specialized NLP tools to assist processing each of the plurality of themes.
Clause 8. The computerized method of generating an RTSM system of Clause 7, wherein the one or more specialized interpreters comprise at least one of a study level interpreter, a primary event interpreter, a randomization interpreter, an action interpreter, a drug supply interpreter, and a notification interpreter.
Clause 9. A Randomization and Trial Supply Management (RTSM) system generator, executed by at least one programmable processor in a computer system, comprising:
   a specification editor for receiving and editing a specification for a clinical trial;
   a specification viewer for presenting the specification and providing feedback to a user;
   a specification checker for checking and improving quality of the specification;
   a specification interpreter configured to utilize natural language processing (LP) to interpret the specification; and
   a study builder configured to build a clinical trial study based on output of the specification interpreter and to configure an RTSM system based on the clinical trial study.
Clause 10. The RTSM system generator of Clause 9, wherein the specification interpreter comprises:
   a master interpreter;
   one or more specialized interpreters;
   one or more information extractors;
   one or more specialized NLP tools; and
   one or more core NLP libraries.
Clause 11. The RTSM system generator of Clause 10, wherein the one or more specialized interpreters comprise at least one of a study level interpreter, a primary event interpreter, a randomization interpreter, an action interpreter, a drug supply interpreter, and a notification interpreter.
Clause 12. A Randomization and Trial Supply Management (RTSM) system with a built-in forecaster, comprising:
   at least one programmable processor; and
   a computer-readable medium storing instructions that, when executed, cause the at least one programmable processor to perform operations comprising:
      receiving at least one supply chain control levels via a user interface of the RTSM system from a user;
      adjusting configuration of the RTSM system based on the at least one supply chain control levels; and
      presenting adjustment result to the user dynamically.
Clause 13. The RTSM system of Clause 12, wherein:
   the at least one supply chain control levels include a first confidence level for unpredictable demand of known and unknown patients; and
   the user interface presents a first user interface (UI) element to receive the first confidence level from the user.
Clause 14. The RTSM system of Clause 13, wherein:
   the at least one supply chain control levels include a second confidence level for demand of patients with a partially known schedule; and
   the user interface presents a second user interface (UI) element to receive the second confidence level from the user.
Clause 15. The RTSM system of Clause 12, wherein:
   the at least one supply chain control levels include a number for a long prediction window; and
   the user interface presents a third user interface (UI) element to receive the number for the long prediction window.
Clause 16. The RTSM system of Clause 12, wherein the forecaster utilizes at least one statistical distributions to perform forecasting.
Clause 17. The RTSM system of Clause 16, wherein the at least one statistical distributions comprise Poisson distribution.

It is to be understood that the disclosed subject matter is not limited in its application to the details of construction and to the arrangements of the components set forth in the following description or illustrated in the drawings. The disclosed subject matter is capable of other embodiments and of being practiced and carried out in various ways. Also, it is to be understood that the phraseology and terminology employed herein are for the purpose of illustration and should not be regarded as limiting.

As such, those skilled in the art will appreciate that the conception, upon which this disclosure is based, may readily be utilized as a basis for the designing of other structures, methods, and systems for carrying out the several purposes of the disclosed subject matter. It is important, therefore, that the clauses and claims be regarded as including such equivalent constructions insofar as they do not depart from the spirit and scope of the disclosed subject matter.

Various implementations of the subject matter described herein may be realized in digital electronic circuitry, integrated circuitry, specially designed ASICs (application specific integrated circuits), computer hardware, firmware, software, and/or combinations thereof. These various implementations may include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and may be implemented in a high-level procedural and/or object-oriented programming language, and/or functional programming language, and/or in assembly/machine language. As used herein, the term "machine-readable medium" refers to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the subject matter described herein may be implemented on a computer having a display device (e.g., a LCD or LED) for displaying information to the user and in some instances a keyboard and a pointing device (e.g., a mouse or a trackball) by which the user may provide input to the computer. In other instances, the subject matter described herein may be implemented on mobile devices such as tablets, phablets, or/and smartphones. Other kinds of devices may be used to provide for interaction with a user as well; for example, feedback provided to the user may be any form of sensory feedback (e.g., visual feedback, auditory feedback, or tactile feedback); and input from the user may be received in any form, including acoustic, speech, or tactile input.

The subject matter described herein may be implemented in a computing system that includes a back-end component (e.g., as a data server), or that includes a middleware component (e.g., an application server), or that includes a front-end component (e.g., a client computer having a graphical user interface or a Web browser through which a user may interact with an implementation of the subject matter described herein), or any combination of such back-end, middleware, or front-end components. The components of the system may be interconnected by any form or medium of digital data communication (e.g., a communication network). Examples of communication networks include a local area network ("LAN"), a wide area network ("WAN"), and the Internet.

The computing system may include clients and servers. A client and server are generally remote from each other and typically interact through a communication network. The relationship of client and server arises by virtue of computer programs running on the respective computers and having a client-server relationship to each other.

Although a few variations have been described in detail above, other modifications are possible. For example, the logic flows depicted in the accompanying figures and described herein do not require the particular order shown, or sequential order, to achieve desirable results. Other embodiments may be within the scope of the clauses and claims.

## Claims

1. A computerized method for generating a Randomization and Trial Supply Management (RTSM) system, performed by at least one programmable processor in a computer system, the method comprising:
generating an instance of a randomization and trial supply management (RTSM) system configured for a clinical trial based on natural language processing (NLP) of an electronic specification describing the clinical trial, wherein the RTSM system is automatically configurable and wherein the generating the instance of the RTSM system based on NLP comprises:
receiving the electronic specification for the clinical trial, wherein the specification comprises natural language text describing the clinical trial;
checking and improving quality of the electronic specification, including:
determining whether the electronic specification includes inconsistent or conflicting usage of one or more terms;
in response to determining that the electronic specification describing the clinical trial includes inconsistent or conflicting usage of the one or more terms,
generating one or more feedback messages regarding the electronic specification and the inconsistent or conflicting usage of the one or more terms; and
receiving an improved electronic specification based on one or more responses to the one or more feedback messages, the improved electronic specification correcting the inconsistent or conflicting usage of the one or more terms;
interpreting the specification utilizing natural language processing, including
dividing the improved electronic specification into one or more sections based on one or more characteristics detected within the electronic specification;
extracting information regarding the clinical trial from each of the one or more sections by applying NLP to the one or more sections of the improved electronic specification, including, for each section,
selecting an NLP interpreter from a plurality of NLP interpreters based on the section and/or characteristics of the section; and
interpreting the section using the selected NLP interpreter; and
generating configuration information for the RTSM system using the extracted information; and
building the instance of the RTSM system based on the configuration information, wherein the built instance of the RTSM system is usable to conduct the clinical trial.

2. The method of claim 1, wherein the one or more feedback messages warn of one or more potential defects in the electronic specification and/or suggest usage of consistent keywords.

3. The method of claim 2, wherein determining whether the electronic specification includes inconsistent or conflicting usage of one or more terms comprises determining whether the electronic specification includes inconsistent or conflicting usage of keywords, phrases, or sentences.

4. The method of any of the preceding claims, wherein:
the electronic specification of the clinical trial includes one or more tables including the information regarding the clinical trial, and
extracting the information regarding the clinical trial comprises applying NLP to the one or more tables to extract the information regarding the clinical trial.

5. The method of any of the preceding claims, wherein extracting the information comprises:
extracting, from each of the one or more sections, information relating to:
a number of patients in the clinical trial;
a schedule of one or more visits by the patients;
supply chain organization, supply shipments, and/or patient kits; or
when and how to notify a user about a study event.

6. The method of any of the preceding claims, wherein the configuration information includes drug supply information.

7. The method of any of the preceding claims, wherein building the instance of the RTSM system comprises configuring the RTSM system to perform acts including:
determining, for a site associated with the clinical trial, whether to replenish one or more supplies for the clinical trial by determining whether an inventory level of the one or more supplies satisfies a threshold amount; and
determining, a quantity of the one or more supplies to replenish for the site in response to determining to replenish the one or more supplies, wherein the quantity is determined based on supply chain timing and one or more expiration requirements of the one or more supplies.

8. The method of any of the preceding claims wherein the information includes notification information, and wherein building the instance of the RTSM system comprises configuring the RTSM system to notify the user about one or more supply chain-related events in the clinical trial.

9. The method of any of the preceding claims, further comprising:
receiving at least one supply chain control level via a user interface of the instance of the RTSM system; and
adjusting a configuration of the instance of the RTSM system based on the at least one supply chain control level.

10. The method of claim 9, wherein:
the at least one supply chain control level includes a first confidence level for unpredictable demand of known and unknown patients; and
the user interface presents a first user interface (UI) element to receive the first confidence level.

11. The method of claim 10, wherein:
the at least one supply chain control level includes a second confidence level for demand of patients with a partially known schedule; and
the user interface presents a second user interface (UI) element to receive the second confidence level.

12. The method of any of the claims 9-11, wherein:
the at least one supply chain control level includes a number for a long prediction window;
and
the user interface presents a third user interface (UI) element to receive the number for the long prediction window.

13. The method of any of the claims 1-12, wherein the forecaster utilizes at least one statistical distribution to perform forecasting.

14. The method of claim 13, wherein the at least one statistical distribution comprises a Poisson distribution.

15. A computer system for generating a Randomization and Trial Supply Management (RTSM) system, comprising:
at least one programmable processor; and
a computer-readable medium storing instructions that, when executed, cause the at least one programmable processor to perform a method according to any of claims 1-14.
